# EUROPEAN PATENT APPLICATION

(11) **EP 2 006 324 A2**
(43) Date of publication of application: **24.12.2008**
(21) Application number: 07739754.5
(22) Date of filing: 27.03.2007
(51) Int. Cl.: C08K 5/3492, C07D 251/24, C08L 69/00, C08L 101/00

(54) **POLYMER MATERIAL COMPOSITION**

(30) Priority: 30.03.2006 JP 2006095273; 30.03.2006 JP 2006095274
(71) Applicant: Adeka Corporation, Arakawa-ku Tokyo 116-0012 (JP)
(72) Inventor: NEGISHI, Yoshinori, Saitama-shi, Saitama 336-0022 (JP); AYABE, Takashi, Saitama-shi, Saitama 336-0022 (JP); SUZUKI, Masato, Saitama-shi, Saitama 336-0022 (JP); TOBITA, Etsuo, Saitama-shi, Saitama 336-0022 (JP)
(74) Representative: Jones, Helen M.M.
(86) International application number: PCT/JP2007/056316
(87) International publication number: WO 2007/114112

(57) **Abstract**

[Object] To provide a polymeric material composition having high heat resistance, excellent compatibility, and greatly improved weatherability.

[Solving Means] A polymeric material composition containing:
a polymeric material and a triazine compound having the general formula (I): 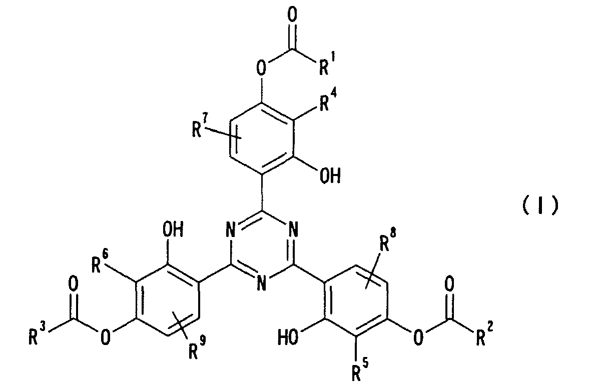 wherein R¹ to R³ denote an alkyl group, a cycloalkyl group, an alkenyl group, an aryl group, an alkylaryl group, or an arylalkyl group, these groups being optionally substituted by a hydroxy group, a halogen atom, a cyano group, a nitro group, an alkyl group, or an alkoxy group, and/or being optionally interrupted by an oxygen atom, a sulfur atom, a carbonyl group, an ester group, an amide group, or an imino group; R⁴ to R⁶ denote an alkyl group or an alkenyl group; and R⁷ to R⁹ denote a hydrogen atom, a halogen atom, an alkyl group, or an alkenyl group.

## Description

### Technical Field

The present invention relates to a polymeric material composition that contains a triazine compound having a particular structure.

### Background Art

Polymeric materials, such as polyethylene, polypropylene, styrene resins, polyvinyl chloride, polycarbonate, and polyesters, organic pigments, and organic dyes may deteriorate, discolor, or become reduced in mechanical strength by the action of light and may be nondurable.

Thus, various ultraviolet absorbers have been used to prevent these organic materials from deteriorating and to control the wavelength of transmitted light. Examples of the ultraviolet absorbers include benzophenones, benzotriazoles, 2,4,6-triaryltriazines, and cyanoacrylates.

Depending on their structures, these ultraviolet absorbers have different ultraviolet absorption spectra, different compatibilities with an organic material, and different volatilities. Thus, different ultraviolet absorbers are used for different applications. In particular, a thin protective layer requires an enhanced ultraviolet-shielding effect and therefore an increased amount of ultraviolet absorber. Thus, there is a need for an ultraviolet absorber that has excellent compatibility with a substrate layer, low volatility, and high absorptivity.

2,4,6-triaryltriazine ultraviolet absorbers have been added and used in polymeric materials. Among 2,4,6-triaryltriazine ultraviolet absorber compounds, especially, a 2,4,6-tris(2-hydroxyaryl)triazine compound, which has a hydroxy group at an ortho position of the aryl group, is reported to have high absorptivity, an absorption wavelength at which an organic material does not have color, and high heat resistance, and may be used in photographic materials and polymeric materials (Patent Documents 1 and 2).

However, the 2,4,6-tris(2-hydroxyaryl)triazine ultraviolet absorber still has low compatibility with polymeric materials, such as polycarbonate and polyolefins, and insufficient heat resistance and weatherability.

Patent Documents 3 and 4 disclose triazine compounds according to the present invention. However, these triazine compounds are exemplified in connection with optical recording materials and optical films. Patent Documents 3 and 4 do not limit the structure of a triazine compound according to the present invention. In addition, Patent Documents 3 and 4 do not disclose that a triazine compound used in the present invention has high heat resistance and excellent compatibility with polymeric materials and imparts excellent weatherability to polymeric materials.
Patent Document 1: Japanese Unexamined Patent Application Publication No. 5-232630
Patent Document 2: Japanese Unexamined Patent Application Publication No. 11-71356
Patent Document 3: Japanese Unexamined Patent Application Publication No. 2004-160883
Patent Document 4: WO 2005/109052

### Disclosure of the Invention

### Problems to be Solved by the Invention

As described above, to date, techniques for improving the weatherability or heat resistance of a polymeric material have not been good enough. Accordingly, it is an object of the present invention to provide a polymeric material composition having high heat resistance, excellent compatibility, and greatly improved weatherability.

### Means for Solving the Problems

As a result of extensive research to solve the above-mentioned problems, the present inventors have focused on a 2,4,6-tris(2-hydroxyaryl)triazine compound having a particular structure and have perfected the present invention by discovering that a polymeric material composition containing this compound can achieve the object.

A polymeric material composition according to the present invention contains a polymeric material and a triazine compound having the general formula (I): 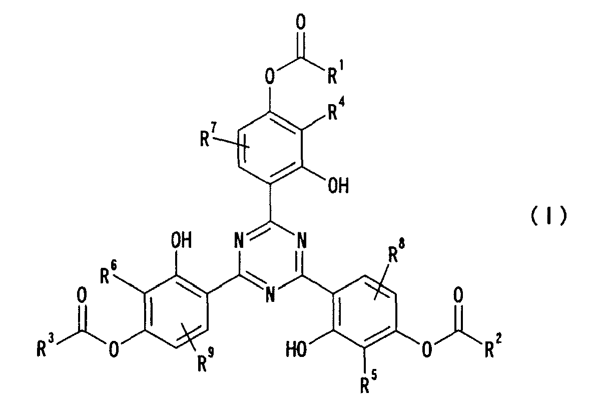 wherein R¹ to R³ independently denote a straight- or branched-chain alkyl group having 1 to 18 carbon atoms, a cycloalkyl group having 3 to 8 carbon atoms, an alkenyl group having 3 to 8 carbon atoms, or an aryl group, an alkylaryl group, or an arylalkyl group each having 6 to 18 carbon atoms. The alkyl group, the cycloalkyl group, the alkenyl group, the aryl group, the alkylaryl group, or the arylalkyl group may be optionally substituted by a hydroxy group, a halogen atom, a cyano group, a nitro group, or an alkyl group or an alkoxy group each having 1 to 18 carbon atoms, and/or may be optionally interrupted by an oxygen atom, a sulfur atom, a carbonyl group, an ester group, an amide group, or an imino group; R⁴ to R⁶ independently denote a hydrogen atom, a straight- or branched-chain alkyl group having 1 to 8 carbon atoms, or an alkenyl group having 3 to 8 carbon atoms; and R⁷ to R⁹ independently denote a hydrogen atom, a halogen atom, a straight- or branched-chain alkyl group having 1 to 8 carbon atoms, or an alkenyl group having 3 to 8 carbon atoms.

A polymeric material composition according to the present invention contains the triazine compound wherein R⁴, R⁵, and R⁶ in the general formula (I) are preferably methyl groups, and R⁷, R⁸, and R⁹ in the general formula (I) are more preferably hydrogen atoms. A polymeric material composition according to the present invention contains the triazine compound wherein R⁴, R⁵, R⁶, R⁷, R⁸, and R⁹ in the general formula (I) are preferably hydrogen atoms.

In a polymeric material composition according to the present invention, the polymeric material is preferably a polycarbonate resin.

### Advantages

The present invention can provide a polymeric material composition that is resistant to coloring in processing and has high heat resistance and excellent weatherability.

### Best Modes for Carrying Out the Invention

Preferred examples of the present invention will be described in detail below.

The triazine compound according to the present invention has the general formula (I): 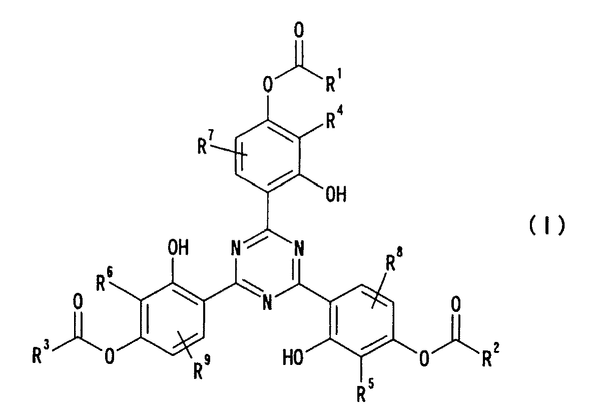 wherein R¹ to R³ independently denote a straight- or branched-chain alkyl group having 1 to 18 carbon atoms, a cycloalkyl group having 3 to 8 carbon atoms, an alkenyl group having 3 to 8 carbon atoms, or an aryl group, an alkylaryl group, or an arylalkyl group each having 6 to 18 carbon atoms. The alkyl group, the cycloalkyl group, the alkenyl group, the aryl group, the alkylaryl group, or the arylalkyl group may be optionally substituted by a hydroxy group, a halogen atom, a cyano group, a nitro group, or an alkyl group or an alkoxy group each having 1 to 18 carbon atoms, and/or may be optionally interrupted by an oxygen atom, a sulfur atom, a carbonyl group, an ester group, an amide group, or an imino group; R⁴ to R⁶ independently denote a hydrogen atom, a straight- or branched-chain alkyl group having 1 to 8 carbon atoms, or an alkenyl group having 3 to 8 carbon atoms; and R⁷ to R⁹ independently denote a hydrogen atom, a halogen atom, a straight- or branched-chain alkyl group having 1 to 8 carbon atoms, or an alkenyl group having 3 to 8 carbon atoms.

In the triazine compound having the general formula (I) according to the present invention, the straight- or branched-chain alkyl group having 18 carbon atoms or less denoted by R¹ to R³ may be methyl, ethyl, propyl, isopropyl, butyl, sec-butyl, tert-butyl, isobutyl, amyl, isoamyl, tert-amyl, hexyl, heptyl, 2-heptyl, isoheptyl, tert-heptyl, n-octyl, isooctyl, tert-octyl, 2-ethylhexyl, nonyl, isononyl, decyl, undecyl, dodecyl, tridecyl, tetradecyl, pentadecyl, hexadecyl, heptadecyl, octadecyl, or the like. The substituted or interrupted straight- or branched-chain alkyl group having 18 carbon atoms or less denoted by R¹ to R³ may be chloromethyl, dichloromethyl, trichloromethyl, 2-hydroxyethyl, 2-hydroxypropyl, 3-hydroxypropyl, 2-methoxyethyl, 2-ethoxyethyl, 2-butoxyethyl, 2-methoxypropyl, 3-methoxypropyl, 2,3-dihydroxypropyl, 2-hydroxy-3-methoxypropyl, 2,3-dimethoxypropyl, 2-(2-methoxyethoxy)ethyl, or the like.

The cycloalkyl group having 3 to 8 carbon atoms denoted by R¹ to R³ may be cyclopropyl, cyclopentyl, cyclohexyl, cycloheptyl, cyclooctyl, or the like.

The alkenyl group having 3 to 8 carbon atoms denoted by R¹ to R³ may be a straight- or branched-chain propenyl, butenyl, pentenyl, hexenyl, heptenyl, or octenyl, each having an unsaturated bond at any position.

The aryl group or alkylaryl group each having 6 to 18 carbon atoms denoted by R¹ to R³ may be phenyl, naphthyl, 2-methylphenyl, 3-methylphenyl, 4-methylphenyl, 4-vinylphenyl, 3-isopropylphenyl, 4-isopropylphenyl, 4-butylphenyl, 4-isobutylphenyl, 4-tert-butylphenyl, 4-hexylphenyl, 4-cyclohexylphenyl, 4-octylphenyl, 4-(2-ethylhexyl)phenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2,4-di-tert-butylphenyl, 2,5-di-tert-butylphenyl, 2,6-di-tert-butylphenyl, 2,4-di-tert-pentylphenyl, 2,5-di-tert-amylphenyl, 2,5-di-tert-octylphenyl, biphenyl, 2,4,5-trimethylphenyl, or the like. The arylalkyl group having 6 to 18 carbon atoms denoted by R¹ to R³ may be benzyl, phenethyl, 2-phenylpropane-2-yl, diphenylmethyl, or the like.

The straight- or branched-chain alkyl group having 1 to 8 carbon atoms denoted by R⁴ to R⁶ may be methyl, ethyl, propyl, isopropyl, butyl, sec-butyl, tert-butyl, isobutyl, amyl, tert-amyl, octyl, tert-octyl, or the like.

The alkenyl group having 3 to 8 carbon atoms denoted by R⁴ to R⁶ may be a straight- or branched-chain propenyl, butenyl, pentenyl, hexenyl, heptenyl, or octenyl, each having an unsaturated bond at any position.

The straight- or branched-chain alkyl group having 1 to 8 carbon atoms denoted by R⁷ to R⁹ may be methyl, ethyl, propyl, isopropyl, butyl, sec-butyl, tert-butyl, isobutyl, amyl, tert-amyl, octyl, tert-octyl, or the like.

The alkenyl group having 3 to 8 carbon atoms denoted by R⁷ to R⁹ may be a straight- or branched-chain propenyl, butenyl, pentenyl, hexenyl, heptenyl, or octenyl, each having an unsaturated bond at any position.

Among the triazine compound having the general formula (I), a triazine compound having the general formula (II) is preferred because of ease of production and low cost. 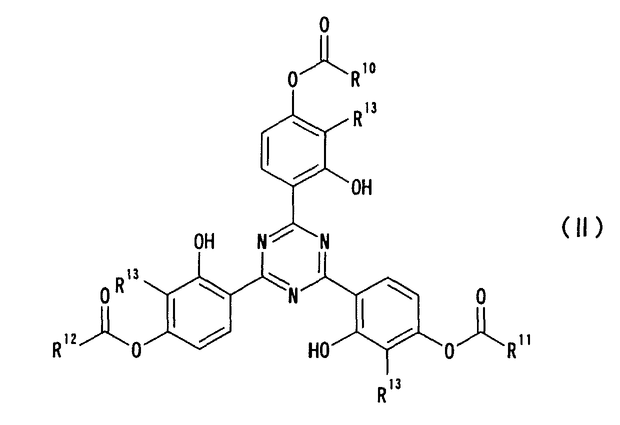 wherein R¹⁰ to R¹² independently denote a straight- or branched-chain alkyl group having 1 to 12 carbon atoms or a cycloalkyl group having 3 to 8 carbon atoms, and R¹³ independently denote a hydrogen atom or a methyl group.

In the triazine compound having the general formula (II), the straight- or branched-chain alkyl group having 1 to 12 carbon atoms denoted by R¹⁰ to R¹² may be methyl, ethyl, propyl, isopropyl, butyl, sec-butyl, tert-butyl, isobutyl, amyl, isoamyl, tert-amyl, hexyl, heptyl, 2-heptyl, isoheptyl, tert-heptyl, n-octyl, isooctyl, tert-octyl, 2-ethylhexyl, nonyl, isononyl, decyl, undecyl, dodecyl, or the like.

The cycloalkyl group having 3 to 8 carbon atoms denoted by R¹⁰ to R¹² may be cyclopropyl, cyclopentyl, cyclohexyl, cycloheptyl, cyclooctyl, or the like.

Specific examples of the triazine compound having the general formula (I) or (II) according to the present invention include the following compounds No. 1 to No. 28. In the chemical formulae described below, Me denotes a methyl group, Et denotes an ethyl group, Pr denotes a propyl group, iPr denotes an isopropyl group, Bu denotes a butyl group, Am denotes an amyl group, iAm denotes an isoamyl group, and cyHx denotes a cyclohexyl group.

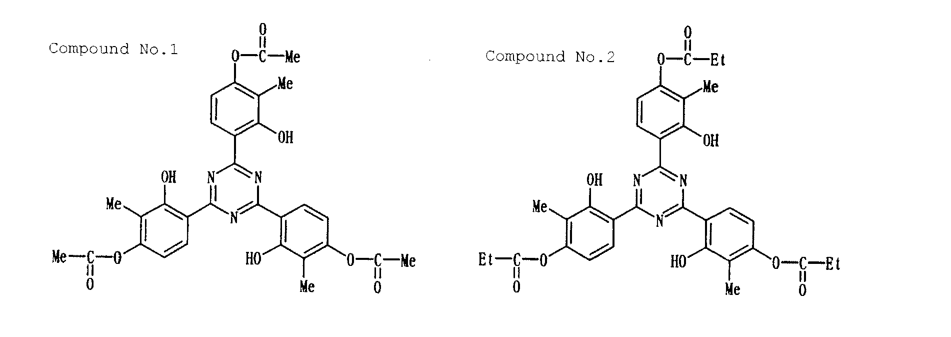

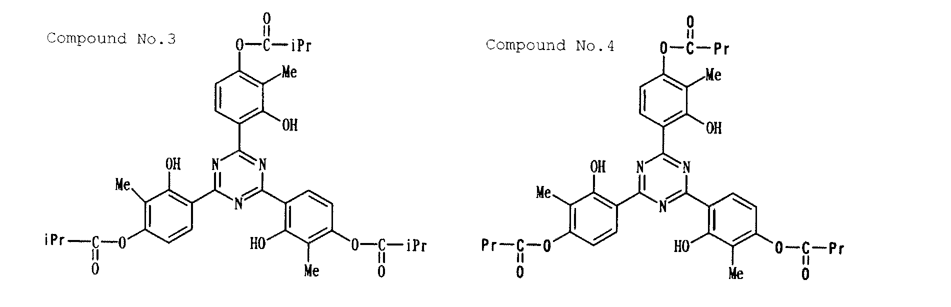

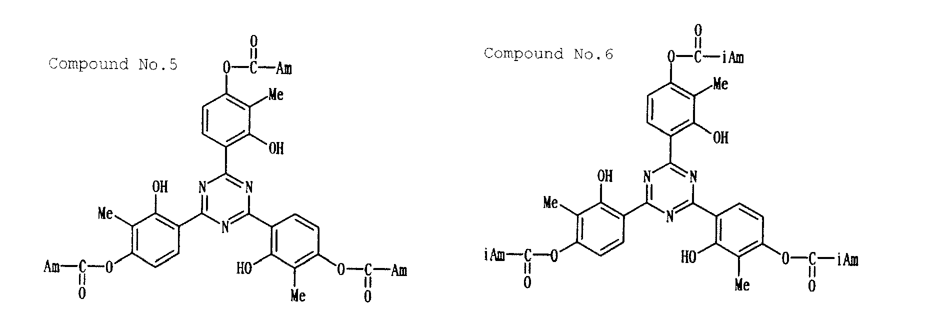

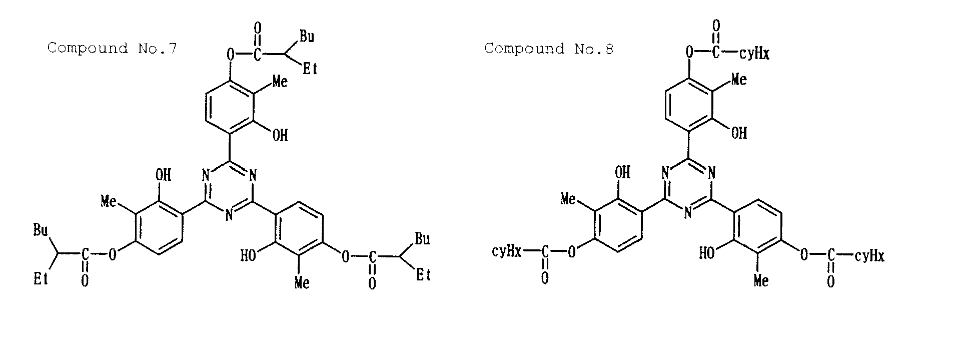

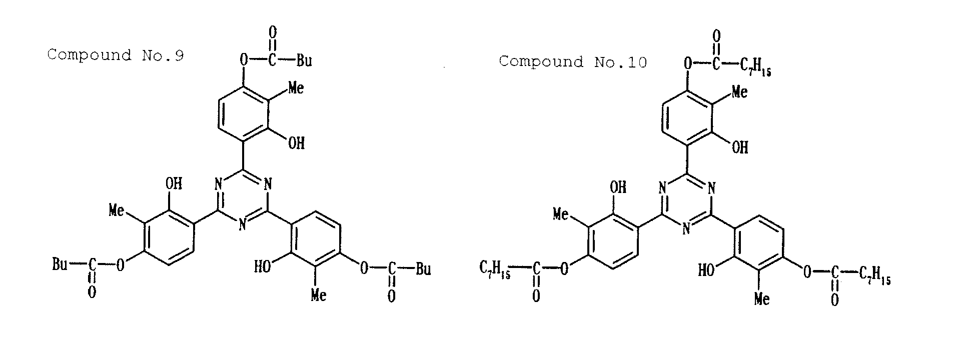

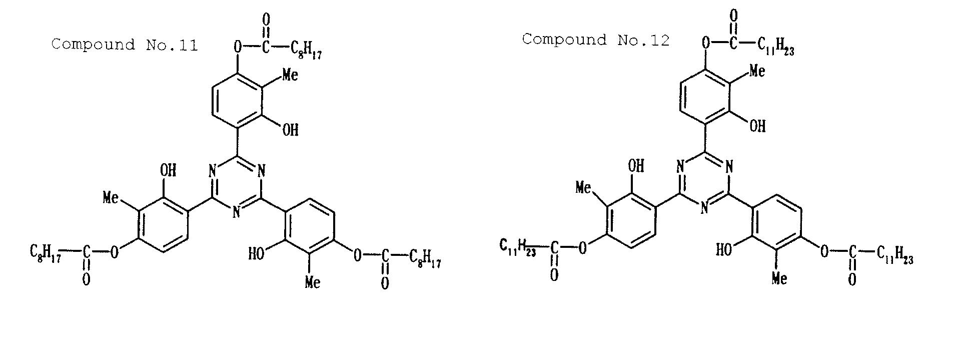

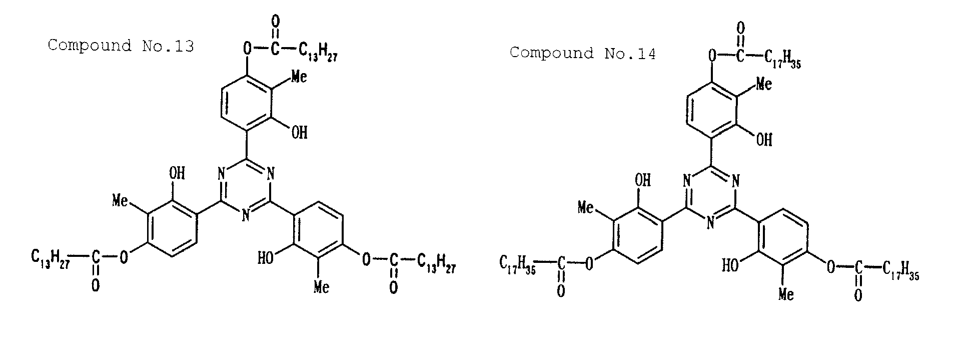

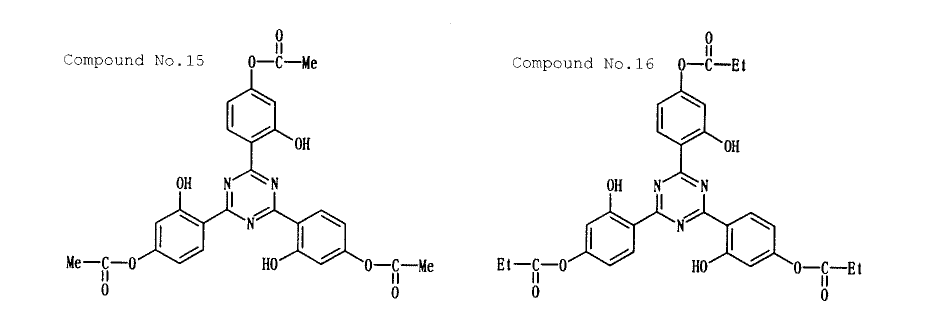

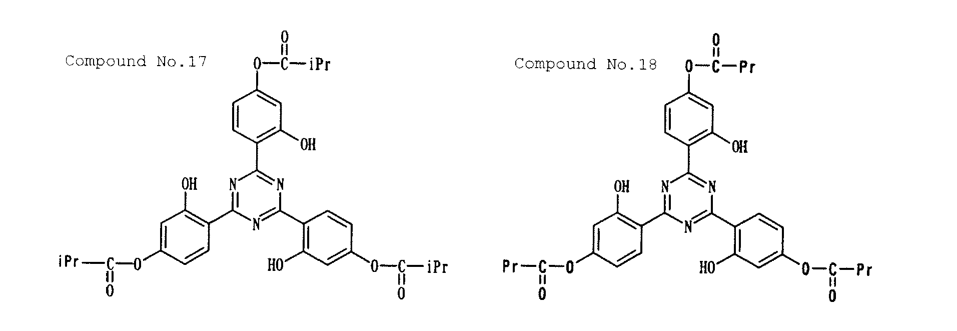

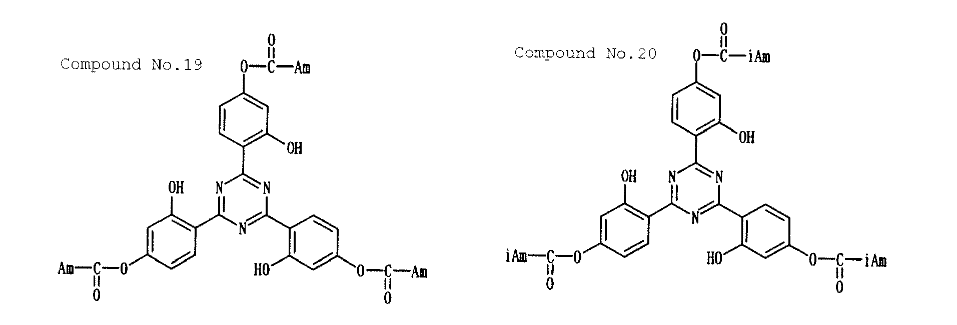

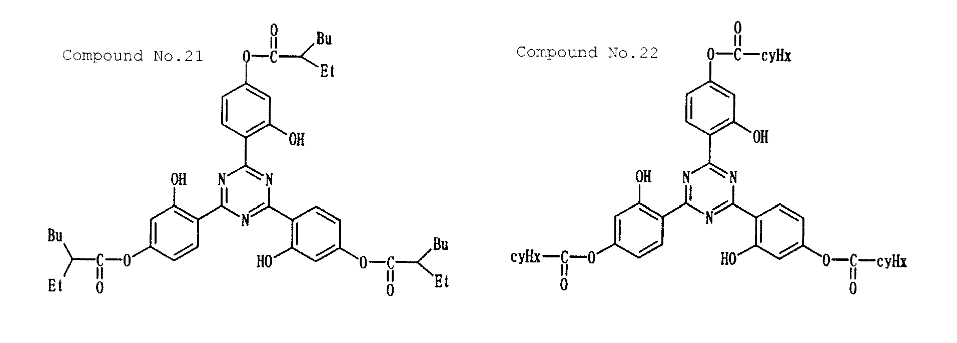

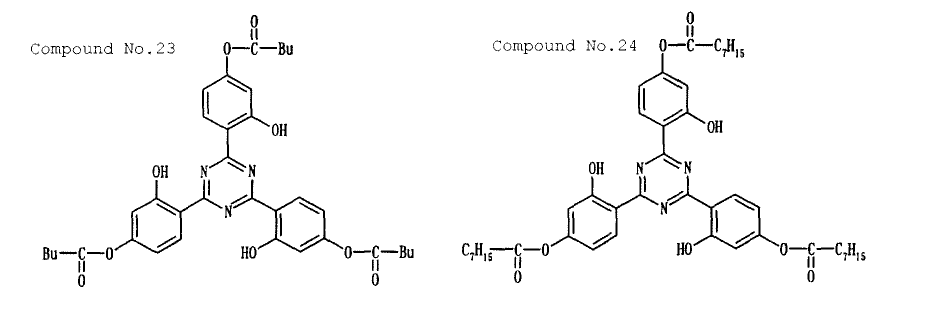

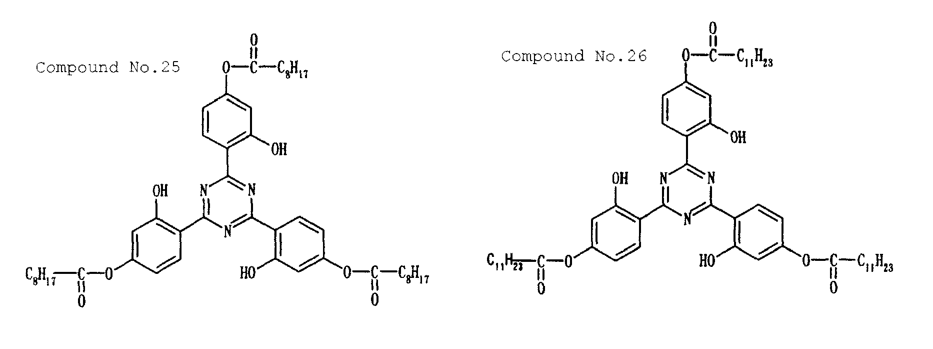

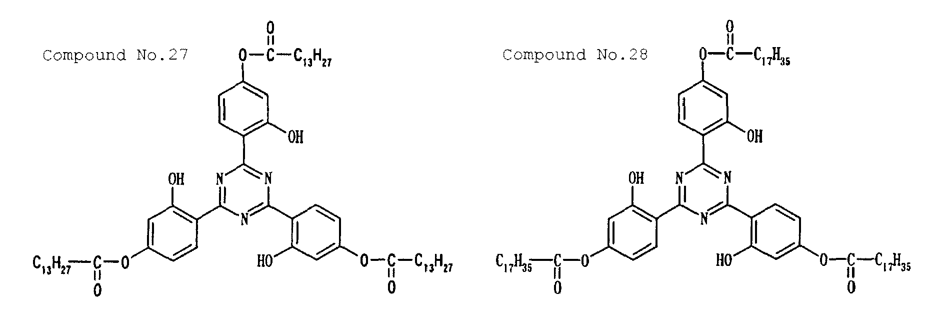

The method of synthesizing the triazine compound having the general formula (I) is not limited, and can apply a common method for synthesizing compounds having the triazine structure. For example, the synthesis method involves an additional reaction between cyanuric chloride and a phenol derivative or a resorcinol derivative using aluminum chloride. The substituents denoted by R¹ to R⁹ may be introduced after the triazine structure is formed, or may be introduced into the phenol derivative or the resorcinol derivative before the triazine structure is formed.

For example, the R¹ to R³ substituents may be introduced by first synthesizing an intermediate having a triazine ring from cyanuric chloride and a phenol derivative or a resorcinol derivative, and then reacting a hydroxyl group at a para position of an aryl group of the intermediate with a carboxylic acid derivative (carboxylic acid, carboxylic acid chloride, a carboxylate ester, or a carboxylate salt) having R¹ to R³ substituents to form an ester bond.

In particular, a triazine compound having the general formula (II) that is produced using 2-methylresorcinol as a raw material is preferred because of ease of synthesis and low cost.

The polymeric material used in the present invention will be described below.

Examples of the polymeric material include thermoplastic resins, thermosetting resins, and elastomers.

The thermoplastic resins include thermoplastic synthetic resins and combinations thereof. Examples of the thermoplastic synthetic resins include polyolefins and copolymers thereof, for example, α-olefin polymers, such as high-density, low-density, and straight-chain low-density polyethylenes, polypropylene, polybutene-1, and poly-3-methylpentene, an ethylene-vinyl acetate copolymer, and an ethylene-propylene copolymer; halogen-containing resins, such as polyvinyl chloride, polyvinylidene chloride, chlorinated polyethylene, chlorinated polypropylene, polyvinylidene fluoride, chlorinated rubber, a vinyl chloride-vinyl acetate copolymer, a vinyl chloride-ethylene copolymer, a vinyl chloride-vinylidene chloride copolymer, a vinyl chloride-vinylidene chloride-vinyl acetate terpolymer, a vinyl chloride-acrylate copolymer, a vinyl chloride-maleate copolymer, and a vinyl chloride-cyclohexylmaleimide copolymer; petroleum resins; cumarone resins; polystyrene; polyvinyl acetate; acrylic resins; copolymers of styrene and/or α-methylstyrene and another monomer (for example, maleic anhydride, phenylmaleimide, methyl methacrylate, butadiene, or acrylonitrile) (for example, acrylonitrile-styrene copolymer (AS) resins, acrylonitrile-butadienestyrene copolymer (ABS) resins, methyl methacrylate-butadiene-styrene copolymer (MBS) resins, and heat-resistant ABS resins); polymethyl methacrylate; polyvinyl alcohol; polyvinyl formal; polyvinyl butyral; straight-chain polyesters, such as polyethylene terephthalate and polytetramethylene terephthalate; polyphenylene oxide; polyamides, such as polycaprolactam and polyhexamethylene adipamide; polycarbonate; branched-chain polycarbonate; polyacetal; polyphenylene sulfide; polyurethane; and cellulose resins. The thermosetting resins include phenolic resins, urea resins, melamine resins, epoxy resins, and unsaturated polyester resins. The elastomers include isoprene rubber, butadiene rubber, acrylonitrile-butadiene copolymer rubber, and styrene-butadiene copolymer rubber.

Examples of the polymeric material also include plant-derived resins and biodegradable resins, such as polylactic acid.

Among these polymeric materials, polymeric materials containing a heteroatom are preferred because of excellent compatibility with the triazine compound having the general formula (I). The polymeric materials containing a heteroatom include polycarbonate; branched-chain polycarbonate; straight-chain polyesters, such as polyethylene terephthalate and polytetramethylene terephthalate; polyamides, such as polycaprolactone and polyhexamethylene adipamide; polyphenylene oxide; polyphenylene sulfide; and poly(meth)acrylates, such as polymethyl (meth)acrylate and polybutyl (meth)acrylate. Among them, polymeric materials having a heteroatom or an aromatic ring in the main chain, such as polycarbonate, polyethylene terephthalate, and polyphenylene oxide, are particularly preferable. A polycarbonate resin is most preferable.

The polymeric material may be used alone or in combination. The combination of polymeric materials may be a composite, such as polycarbonate/ABS or polycarbonate/polyester, or a laminate of a polycarbonate film and a polycarbonate or poly(meth)acrylate film containing a particular triazine compound having the general formula (I) according to the present invention.

Preferably, a polymeric material composition according to the present invention contains 0.001 to 10 parts by mass, particularly 0.005 to 10 parts by mass, of the triazine compound having the general formula (I) per 100 parts by mass of the polymeric material. This is because sufficiently improved weatherability and improved coloring are achieved in these ranges.

The triazine compound having the general formula (I) according to the present invention may be applied to the polymeric material by any method. The triazine compound may be added in the form of powder, aqueous dispersion, such as emulsion or suspension, or solution in an organic solvent. However, the addition of the triazine compound either during polymerization of the polymeric material or under the condition where a (meth)acryloyl group reacts due to a processing temperature results in significant coloring of the resulting polymeric material composition and is therefore not practical.

If necessary, a polymeric material composition according to the present invention may contain another additive agent, such as a general-purpose antioxidant or a stabilizer, in addition to the triazine compound having the general formula (I). Particularly preferred examples of the additive agent include phenolic, sulfur-containing, and phosphite antioxidants and hindered amine light stabilizers (HALSs). In particular, the hindered amine light stabilizers (HALSs), as typified by a 2,2,6,6-tetramethylpiperidine compound, are preferred because of a synergistic effect with the triazine compound according to the present invention.

The HALSs include a compound having the general formula (III), cyanuric chloride condensed HALSs, and high molecular weight HALSs. 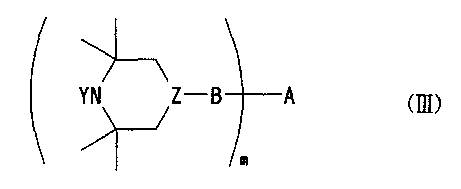 wherein, m denotes an integer in the range of 1 to 6, A denotes a hydrogen atom, an m-valent hydrocarbon group having 1 to 18 carbon atoms, an m-valent acyl group, or an m-valent carbamoyl group, B denotes an oxygen atom, -NH-, or -NRe-, wherein Re denotes an alkyl group having 1 to 8 carbon atoms, Y denotes a hydrogen atom, an oxy radical (·O), an alkoxy group having 1 to 18 carbon atoms, an alkyl group having 1 to 8 carbon atoms, or a hydroxyl group, and Z denotes methine or a group having the general formula (IV): 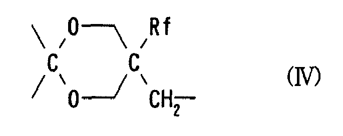 wherein Rf denotes an alkyl group having 1 to 8 carbon atoms.

Examples of the m-valent hydrocarbon group having 1 to 18 carbon atoms denoted by A in the general formula (III) include hydrocarbon compound-derived groups (alkyl, alkanediyl, alkanetriyl, alkanetetrayl, alkanepentayl, and alkanehexayl groups), such as methane, ethane, propane, butane, sec-butane, tert-butane, isobutane, pentane, isopentane, tert-pentane, hexane, cyclohexane, heptane, isoheptane, tert-heptane, n-octane, isooctane, tert-octane, 2-ethylhexane, nonane, isononane, decane, dodecane, tridecane, tetradecane, pentadecane, hexadecane, heptadecane, and octadecane.

The m-valent acyl group denoted by A in the general formula (III) is a carboxylic acid-derived group, an m-valent carboxylic acid-derived group, or an alkyl ester-derived group that is derived from an n-valent carboxylic acid and contains m carboxyl groups and (n-m) ester groups (the carboxylic acid, the m-valent carboxylic acid, and the alkyl ester are referred to as acyl derivatives).

Examples of the acyl derivatives include acetic acid, benzoic acid, 4-trifluoromethylbenzoic acid, salicylic acid, acrylic acid, methacrylic acid, oxalic acid, malonic acid, succinic acid, glutaric acid, adipic acid, pimelic acid, suberic acid, azelaic acid, sebacic acid, dodecanedioic acid, 2-methylsuccinic acid, 2-methyladipic acid, 3-methyladipic acid, 3-methylpentanedioic acid, 2-methyloctanedioic acid, 3,8-dimethyldecanedioic acid, 3,7-dimethyldecanedioic acid, hydrogenated dimer acid, dimer acid, phthalic acid, terephthalic acid, isophthalic acid, naphthalenedicarboxylic acid, cyclohexanedicarboxylic acid, trimellitic acid, pyromellitic acid, trimesic acid, propane-1,2,3-tricarboxylic acid, propane-1,2,3-tricarboxylic acid mono-and di-alkyl esters, pentane-1,3,5-tricarboxylic acid, pentane-1,3,5-tricarboxylic acid mono- and di-alkyl esters, butane-1,2,3,4-tetracarboxylic acid, butane-1,2,3,4-tetracarboxylic acid mono-, di-, and tri-alkyl esters, pentane-1,2,3,4,5-pentacarboxylic acid, pentane-1,2,3,4,5-pentacarboxylic acid mono-, di-, tri-, and tetra-alkyl esters, hexane-1,2,3,4,5,6-hexacarboxylic acid, and hexane-1,2,3,4,5,6-hexacarboxylic acid mono-, di-, tri-, tetra-, and penta-alkyl esters.

The m-valent carbamoyl group denoted by A in the general formula (III) is an isocyanate compound-derived monoalkylcarbamoyl or dialkylcarbamoyl group.

Examples of the isocyanate compound from which the monoalkylcarbamoyl group is derived include tolylene diisocyanate, diphenylmethane-4,4'-diisocyanate, p-phenylene diisocyanate, xylylene diisocyanate, 1,5-naphthylene diisocyanate, 3,3'-dimethyldiphenyl-4,4'-diisocyanate, dianisidine diisocyanate, tetramethylxylylene diisocyanate, isophorone diisocyanate, dicyclohexylmethane-4,4'-diisocyanate, trans-1,4-cyclohexyl diisocyanate, norbornene diisocyanate, 1,6-hexamethylene diisocyanate, 2,2,4(2,2,4)-trimethylhexamethylene diisocyanate, lysine diisocyanate, triphenylmethane triisocyanate, 1-methylbenzol-2,4,6-triisocyanate, and dimethyltriphenylmethane tetraisocyanate.

Examples of the dialkylcarbamoyl group include a diethylcarbamoyl group, a dibutylcarbamoyl group, a dihexylcarbamoyl group, and a dioctylcarbamoyl group.

The m-valent hydrocarbon group having 1 to 18 carbon atoms, the m-valent acyl group, and the m-valent carbamoyl group may be substituted by a halogen atom, a hydroxyl group, an alkyl group, an alkoxy group, a nitro group, or a cyano group.

Examples of the alkyl group having 1 to 8 carbon atoms denoted by Re in the group B in the general formula (III) include methyl, ethyl, propyl, isopropyl, butyl, sec-butyl, tert-butyl, isobutyl, amyl, isoamyl, tert-amyl, hexyl, cyclohexyl, heptyl, isoheptyl, tert-heptyl, 1-ethylpentyl, n-octyl, isooctyl, tert-octyl, and 2-ethylhexyl.

Examples of the alkoxy group having 1 to 18 carbon atoms denoted by Y in the general formula (III) include methoxy, ethoxy, propoxy, isopropoxy, butoxy, sec-butyloxy, tert-butyloxy, isobutyloxy, amyloxy, isoamyloxy, hexyloxy, heptyloxy, octyloxy, 2-ethylhexyloxy, nonyloxy, isononyloxy, decyloxy, dodecyloxy, tridecyloxy, tetradecyloxy, pentadecyloxy, hexadecyloxy, heptadecyloxy, and octadecyloxy. Examples of the alkyl group having 1 to 8 carbon atoms denoted by Y include the same groups as described for Re.

Examples of the alkyl group having 1 to 8 carbon atoms denoted by Rf in the general formula (IV) in the group Z in the general formula (III) include the same groups as described for Re.

Specific examples of the compound having the general formula (III) include 2,2,6,6-tetramethyl-4-piperidylstearate, 1,2,2,6,6-pentamethyl-4-piperidylstearate, 2,2,6,6-tetramethyl-4-piperidylbenzoate, bis(2,2,6,6-tetramethyl-4-piperidyl)sebacate, bis(1,2,2,6,6-pentamethyl-4-piperidyl)sebacate, bis(1-octoxy-2,2,6,6-tetramethyl-4-piperidyl)sebacate, 1,2,2,6,6-pentamethyl-4-piperidylmethacrylate, 2,2,6,6-tetramethyl-piperidylmethacrylate, tetrakis(2,2,6,6-tetramethyl-4-piperidyl)-1,2,3,4-butane tetracarboxylate, tetrakis(1,2,2,6,6-pentamethyl-4-piperidyl)-1,2,3,4-butane tetracarboxylate, bis(2,2,6,6-tetramethyl-4-piperidyl)-bis(tridecyl)-1,2,3,4-butane tetracarboxylate, bis(1,2,2,6,6-pentamethyl-4-piperidyl)·bis(tridecyl)-1,2,3,4-butane tetracarboxylate, bis(1,2,2,6,6-pentamethyl-4-piperidyl)-2-butyl-2-(3,5-di-tert-butyl-4-hydroxybenzyl)malonate, 3,9-bis[1,1-dimethyl-2-[tris(2,2,6,6-tetramethyl-4-piperidyloxycarbonyloxy)butylcarbonyloxy]ethyl]-2,4,8,10-tetraoxaspiro[5.5]undecane, and 3,9-bis[1,1-dimethyl-2-[tris(1,2,2,6,6-pentamethyl-4-piperidyloxycarbonyloxy)butylcarbonyloxy]ethyl]-2,4,8,10-tetraoxaspiro[5.5]undecane.

The cyanuric chloride condensed HALSs for use in a polymeric material composition according to the present invention include a 1,6-bis(2,2,6,6-tetramethyl-4-piperidylamino)hexane/2,4-dichloro-6-morpholino-s-triazine polycondensate, a 1,6-bis(2,2,6,6-tetramethyl-4-piperidylamino)hexane/2,4-dichloro-6-tert-octylamino-s-triazine polycondensate, 1,5,8,12-tetrakis[2,4-bis(N-butyl-N-(2,2,6,6-tetramethyl-4-piperidyl)amino)-s-triazine-6-yl]-1,5,8,12-tetraazadodecane, 1,5,8,12-tetrakis[2,4-bis(N-butyl-N-(1,2,2,6,6-pentamethyl-4-piperidyl)amino)-s-triazine-6-yl]-1,5,8,12-tetraazadodecane, 1,6,11-tris[2,4-bis(N-butyl-N-(2,2,6,6-tetramethyl-4-piperidyl)amino)-s-triazine-6-ylamino]undecane, and 1,6,11-tris[2,4-bis(N-butyl-N-(1,2,2,6,6-pentamethyl-4-piperidyl)amino)-s-triazine-6-ylamino]undecane.

The high molecular weight HALSs for use in a polymeric material composition according to the present invention include a 1-(2-hydroxyethyl)-2,2,6,6-tetramethyl-4-piperidinol/diethyl succinate polycondensate and a 1,6-bis(2,2,6,6-tetramethyl-4-piperidylamino)hexane/dibromoethane polycondensate.

Examples of the phosphite antioxidant for use in a polymeric material composition according to the present invention include triphenyl phosphite, tris(2,4-di-tert-butylphenyl)phosphite, tris(2,5-di-tert-butylphenyl)phosphite, tris(nonylphenyl)phosphite, tris(dinonylphenyl)phosphite, tris(mono/di-mixed nonylphenyl)phosphite, diphenyl acid phosphite, 2,2'-methylenebis(4,6-di-tert-butylphenyl) octyl phosphite, diphenyl decyl phosphite, diphenyl octyl phosphite, di(nonylphenyl)pentaerythritol diphosphite, phenyl diisodecyl phosphite, tributyl phosphite, tris(2-ethylhexyl)phosphite, tridecyl phosphite, trilauryl phosphite, dibutyl acid phosphite, dilauryl acid phosphite, trilauryl trithiophosphite, bis(neopentyl glycol)·1,4-cyclohexanedimethyl diphosphite, bis(2,4-di-tert-butylphenyl)pentaerythritol diphosphite, bis(2,5-di-tert-butylphenyl)pentaerythritol diphosphite, bis(2,6-di-tert-butyl-4-methylphenyl)pentaerythritol diphosphite, bis(2,4-dicumylphenyl)pentaerythritol diphosphite, distearyl pentaerythritol diphosphite, tetra(C12-15 mixed alkyl)-4,4'-isopropylidene diphenyl phosphite, bis[2,2'-methylenebis(4,6-diamylphenyl)]·isopropylidene diphenyl phosphite, tetratridecyl·4,4'-butylidene bis(2-tert-butyl-5-methylphenol)diphosphite, hexa(tridecyl)·1,1,3-tris(2-methyl-5-tert-butyl-4-hydroxyphenyl)butane·triphosphite, tetrakis(2,4-di-tert-butylphenyl)biphenylene diphosphonite, tris(2-[(2,4,7,9-tetrakis-tert-butyldibenzo[d,f][1,3,2]dioxaphosphepin-6-yl)oxy]ethyl)amine, 9,10-dihydro-9-oxa-10-phosphaphenanthrene-10-oxide, and 2-butyl-2-ethylpropanediol·2,4,6-tri-tert-butylphenol monophosphite.

Examples of the phenolic antioxidant for use in a polymeric material composition according to the present invention include 2,6-di-tert-butyl-p-cresol, 2,6-diphenyl-4-octadecyloxyphenol, stearyl(3,5-di-tert-butyl-4-hydroxyphenyl)propionate, distearyl(3,5-di-tert-butyl-4-hydroxybenzyl)phosphonate, tridecyl·3,5-di-tert-butyl-4-hydroxybenzylthioacetate, thiodiethylene bis[(3,5-di-tert-butyl-4-hydroxyphenyl)propionate], 4,4'-thiobis(6-tert-butyl-m-cresol), 2-octylthio-4,6-di(3,5-di-tert-butyl-4-hydroxyphenoxy)-s-triazine, 2,2'-methylene bis(4-methyl-6-tert-butylphenol), bis[3,3-bis(4-hydroxy-3-tert-butylphenyl)butyric acid]glycol ester, 4,4'-butylidene bis(2,6-di-tert-butylphenol), 4,4'-butylidene bis(6-tert-butyl-3-methylphenol), 2,2'-ethylidene bis(4,6-di-tertbutylphenol), 1,1,3-tris(2-methyl-4-hydroxy-5-tert-butylphenyl)butane, bis[2-tert-butyl-4-methyl-6-(2-hydroxy-3-tert-butyl-5-methylbenzyl)phenyl]terephthalate, 1,3,5-tris(2,6-dimethyl-3-hydroxy-4-tert-butyl benzyl)isocyanurate, 1,3,5-tris(3,5-di-tert-butyl-4-hydroxybenzyl)isocyanurate, 1,3,5-tris(3,5-di-tert-butyl-4-hydroxybenzyl)-2,4,6-trimethylbenzene, 1,3,5-tris[(3,5-di-tert-butyl-4-hydroxyphenyl)propionyloxyethyl]isocyanurate, tetrakis[methylene-3-(3',5'-di-tert-butyl-4'-hydroxyphenyl)propionate]methane, 2-tert-butyl-4-methyl-6-(2-acryloyloxy-3-tert-butyl-5-methylbenzyl)phenol, 3,9-bis[2-(3-tert-butyl-4-hydroxy-5-methylhydrocinnamoyloxy)-1,1-dimethylethyl]-2,4,8,10-tetraoxaspiro[5.5]undecane, and triethylene glycol bis[β-(3-tert-butyl-4-hydroxy-5-methylphenyl)propionate].

Examples of the sulfur-containing antioxidant for use in a polymeric material composition according to the present invention include dialkylthiodipropionates, such as dilauryl esters, dimyristyl esters, myristyl stearyl esters, and distearyl esters of thiodipropionic acid, and β-alkylmercaptopropionic acid esters of polyols, such as pentaerythritol tetra(β-dodecylmercapto propionate).

If necessary, a polymeric material composition according to the present invention may further contain a known ultraviolet absorber other than the triazine compound having the general formula (I), without compromising the advantages of the present invention.

Examples of the ultraviolet absorber for use in a polymeric material composition according to the present invention include 2-hydroxybenzophenones, such as 2,4-dihydroxybenzophenone, 2-hydroxy-4-methoxybenzophenone, 2-hydroxy-4-octoxybenzophenone, and 5,5'-methylenebis(2-hydroxy-4-methoxybenzophenone); 2-(2-hydroxyphenyl)benzotriazoles, such as 2-(2-hydroxy-5-methylphenyl)benzotriazole, 2-(2-hydroxy-5-tert-octylphenyl)benzotriazole, 2-(2-hydroxy-3,5-di-tert-butylphenyl)-5-chlorobenzotriazole, 2-(2-hydroxy-3-tert-butyl-5-methylphenyl)-5-chlorobenzotriazole, 2-(2-hydroxy-3,5-dicumylphenyl)benzotriazole, 2,2'-methylenebis(4-tert-octyl-6-benzotriazolylphenol), a polyethylene glycol ester of 2-(2-hydroxy-3-tert-butyl-5-carboxyphenyl)benzotriazole, 2-[2-hydroxy-3-(2-acryloyloxyethyl)-5-methylphenyl]benzotriazole, 2-[2-hydroxy-3-(2-methacryloyloxyethyl)-5-tert-butylphenyl]benzotriazole, 2-[2-hydroxy-3-(2-methacryloyloxyethyl)-5-tert-octylphenyl]benzotriazole, 2-[2-hydroxy-3-(2-methacryloyloxyethyl)-5-tert-butylphenyl]-5-chlorobenzotriazole, 2-[2-hydroxy-5-(2-methacryloyloxyethyl)phenyl]benzotriazole, 2-[2-hydroxy-3-tert-butyl-5-(2-methacryloyloxyethyl)phenyl]benzotriazole, 2-[2-hydroxy-3-tert-amyl-5-(2-methacryloyloxyethyl)phenyl]benzotriazole, 2-[2-hydroxy-3-tert-butyl-5-(3-methacryloyloxypropyl)phenyl]-5-chlorobenzotriazole, 2-[2-hydroxy-4-(2-methacryloyloxymethyl)phenyl]benzotriazole, 2-[2-hydroxy-4-(3-methacryloyloxy-2-hydroxypropyl)phenyl]benzotriazole, and 2-[2-hydroxy-4-(3-methacryloyloxypropyl)phenyl]benzotriazole; 2-(2-hydroxyphenyl)-4,6-diaryl-1,3,5-triazines, such as 2-(2-hydroxy-4-methoxyphenyl)-4,6-diphenyl-1,3,5-triazine, 2-(2-hydroxy-4-hexyloxyphenyl)-4,6-diphenyl-1,3,5-triazine, 2-(2-hydroxy-4-octoxyphenyl)-4,6-bis(2,4-dimethylphenyl)-1,3,5-triazine, 2-[2-hydroxy-4-(3-C12/C13 mixed alkoxy-2-hydroxypropoxy)phenyl]-4,6-bis(2,4-dimethylphenyl)-1,3,5-triazine, 2-[2-hydroxy-4-(2-acryloyloxyethoxy)phenyl]-4,6-bis(4-methylphenyl)-1,3,5-triazine, 2-(2,4-dihydroxy-3-allylphenyl)-4,6-bis(2,4-dimethylphenyl)-1,3,5-triazine, and 2,4,6-tris(2-hydroxy-3-methyl-4-hexyloxyphenyl)-1,3,5-triazine; benzoates, such as phenyl salicylate, resorcinol monobenzoate, 2,4-di-tert-butylphenyl-3,5-di-tert-butyl-4-hydroxybenzoate, octyl(3,5-di-tert-butyl-4-hydroxy)benzoate, dodecyl(3,5-di-tert-butyl-4-hydroxy)benzoate, tetradecyl(3,5-di-tert-butyl-4-hydroxy)benzoate, hexadecyl(3,5-di-tert-butyl-4-hydroxy)benzoate, octadecyl(3,5-di-tert-butyl-4-hydroxy)benzoate, and behenyl(3,5-di-tert-butyl-4-hydroxy)benzoate; substituted oxanilides, such as 2-ethyl-2'-ethoxyoxanilide, 2-ethoxy-4'-dodecyloxanilide; cyanoacrylates, such as ethyl-α-cyano-β,β-diphenyl acrylate and methyl-2-cyano-3-methyl-3-(p-methoxyphenyl)acrylate; and salts or chelates of metals, such as nickel and chromium.

If necessary, a polymeric material composition according to the present invention may contain an additive agent, for example, an antistatic agent, such as a cationic surfactant, an anionic surfactant, a nonionic surfactant, or an amphoteric surfactant; a flame retardant, such as a halogen compound, a phosphate compound, a phosphoric amide compound, a melamine compound, a melamine salt compound of polyphosphoric acid, a fluorocarbon resin, or a metal oxide; a lubricant, such as a hydrocarbon, a fatty acid, an aliphatic alcohol, an aliphatic ester, an aliphatic amide, or a metal soap; a heavy metal deactivator; hydrotalcite; an organic carboxylic acid; a coloring agent, such as dye or pigment; a processing aid, such as a polyolefin powder; a silicic acid additive agent, such as fumed silica, fine silica particles, silica stone, diatomaceous earth, clay, kaolin, silica gel, calcium silicate, sericite, kaolinite, flint, a feldspar powder, vermiculite, attapulgite, talc, mica, minnesotaite, or pyrophyllite; filler, such as calcium carbonate; a nucleating agent; an organic tin compound; an aliphatic organic acid metal salt, such as magnesium stearate, calcium stearate, or zinc stearate; a plasticizer; an epoxy compound; or a foaming agent.

While the uses of a polymeric material composition according to the present invention are not limited, it can be used as a molded product or a thin film, and is particularly useful as a thin film. Examples of the thin film include synthetic resin films, sheets, and paints, as well as fibers for use in nonwoven fabrics and fabrics.

Specific uses include automotive resin components, such as bumpers, dashboards, and instrument panels; resin components for use in household electrical appliances, such as refrigerators, washing machines, and cleaners; household utensils, such as dishes, buckets, and bath products; resin components for connection, such as connectors; sundry goods, such as toys; medical molded products, such as medical packages, syringes, catheters, and medical tubes; construction materials, such as wall materials, floorings, window frames, and wallpapers; wire coating materials; agricultural materials, such as greenhouses and tunnels; molded products, including films and sheets, such as food packaging materials, for example, wrap and trays; fibers; paints; and coating materials.

### EXAMPLES

The present invention will be further described below with examples and comparative examples. However, the present invention is not limited to these examples.

### SYNTHESIS EXAMPLE 1

### Synthesis of compound No. 7

### (1) Synthesis of intermediate A

An intermediate A having the following formula was synthesized. In a flask, 84.5 mmol of aluminum chloride was slowly added to 65.0 mmol of cyanuric chloride, 278 mmol of 2-methylresorcinol, and 80 ml of chlorobenzene. The reaction system was allowed to react at 80°C for two hours. The reaction system was then cooled to 25°C. After 14 ml of 6 mol/liter aqueous hydrochloric acid was added dropwise, the reaction system was stirred for one hour. The resulting crystal was filtered off and washed with water. Recrystallization with a dimethylformamide solvent produced crystals of the intermediate A in 68.0% yield.

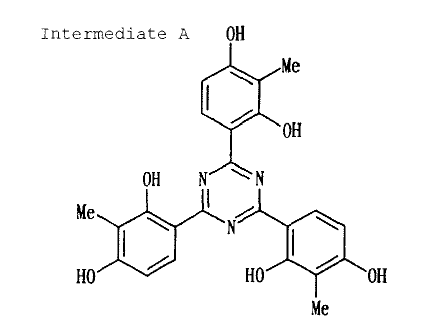

### (2) Synthesis of compound No. 7

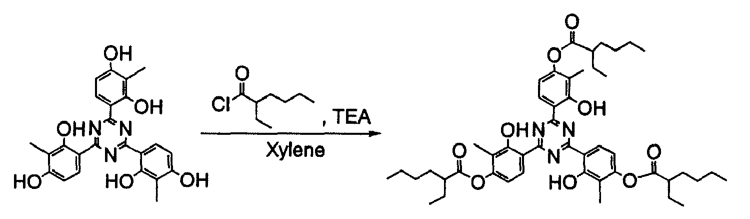

5.0 grams (11.1 x 10⁻³ mol) of the resulting intermediate A, that is, 2,4,6-tri(3-methyl-2,4-dihydroxyphenyl)-1,3,5-triazine, 6.78 g (33.5 x 10⁻³ mol) of triethylamine (TEA), and 18 g of xylene were charged in a 100 mL four-neck round-bottom flask equipped with a mechanical stirrer, a Dimroth condenser, a thermometer, and a nitrogen supply unit. After the reaction system was heated to 80°C, 5.42 g (33.5 x 10⁻³ mol) of 2-ethylhexanoic acid chloride was added dropwise over 30 minutes. The reaction system was allowed to react at 80°C for 6 hours. The reaction rate was measured with an HPLC (using a PEGASIL ODS column manufactured by Senshu Scientific co., ltd. and tetrahydrofuran (THF):water = 3:1 as a developing solvent). After the raw materials were consumed, the reaction mixture was cooled and neutralized with 1 N HCl. The resulting organic phase was washed twice with water and was dried over anhydrous magnesium sulfate. The solvent was then evaporated under reduced pressure. The residue was dispersed in hexane and was filtered. The solvent was then removed from the resulting filtrate to produce 6.4 g of the target compound (compound No. 7) in 70% yield.

The resulting compound was subjected to various analyses. The analyses of the product were as follows:
Melting point: 74.0°C
Infrared absorption spectrum (KBr, cm⁻¹): 3433, 2963, 2932, 2862, 1759, 1605, 1539, 1435, 1308, and 1103
¹H-NMR (90 MHz, CDCl₃, ppm): 13.29 (s, -OH), 8.40 (d, Ph), 6.73 (d, Ph), 2.58 (quin), 2.24 (s, -CH₃), and 2.17-0.95 (m)

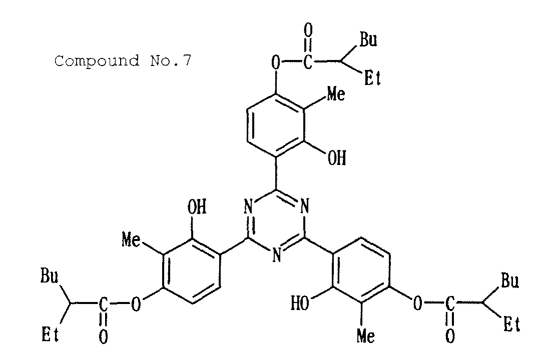

### SYNTHESIS EXAMPLE 2

### Synthesis of compound No. 21

### (1) Synthesis of intermediate B

An intermediate B having the following formula was synthesized. In a flask, 84.5 mmol of aluminum chloride was slowly added to 65.0 mmol of cyanuric chloride, 278 mmol of resorcinol, and 80 ml of chlorobenzene. The mixture was allowed to react at 80°C for two hours. The reaction system was then cooled to 25°C. After 14 ml of 6 mol/liter aqueous hydrochloric acid was added dropwise, the reaction system was stirred for one hour. The resulting crystal was filtered off and washed with water. Recrystallization with a dimethylformamide solvent produced crystals of the intermediate B in 68.0% yield.

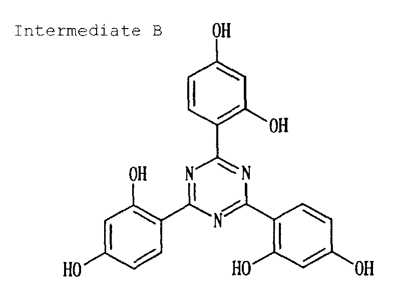

### (2) Synthesis of compound No. 21

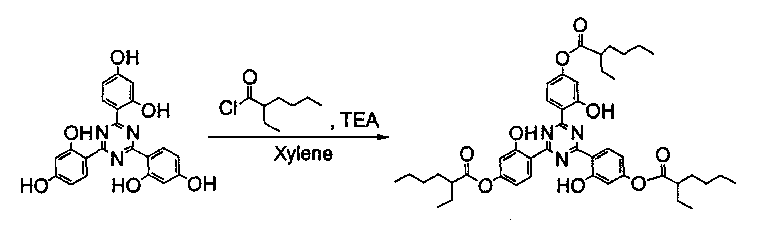

10.0 grams (24.7 x 10⁻³ mol) of the resulting intermediate B, that is, 2,4,6-tri(2,4-dihydroxyphenyl)-1,3,5-triazine, 17.5 g (172.7 x 10⁻³ mol) of triethylamine (TEA), and 70 g of xylene were charged in a 300 mL four-neck round-bottom flask equipped with a mechanical stirrer, a Dimroth condenser, a thermometer, and a nitrogen supply unit. Subsequently, 14.1 g (86.4 x 10⁻³ mol) of 2-ethylhexanoic acid chloride was added dropwise at room temperature over 30 minutes. The reaction system was allowed to react at 50°C for 10 hours. The reaction rate was measured with an HPLC (using a Shodex KF-802 column manufactured by Showa Denko K.K. and tetrahydrofuran (THF) as a developing solvent). After the raw materials were consumed, the reaction mixture was cooled. Ethyl acetate was added to the reaction mixture. The reaction mixture was then neutralized with 1 N HCl. The organic phase was washed twice with water, and the solvent was evaporated under reduced pressure. The residue was dispersed in xylene and was filtered. The resulting filtrate was subjected to silica gel column chromatography (solvent: toluene:hexane = 1:1) to produce 8.7 g of the target compound (compound No. 21) in 45% yield.

The resulting compound was subjected to various analyses. The analyses of the product were as follows:
Infrared absorption spectrum (KBr, cm⁻¹): 3433, 2931, 2873, 1762, 1601, 1504, 1458, 1246, 1146, and 1107
¹H-NMR (90 MHz, CDCl₃, ppm): 12.96 (s, -OH), 8.20 (d, Ph), 6.88 (s, Ph), 6.81 (d, Ph), 2.55 (quin), and 1.85-0.95 (m)

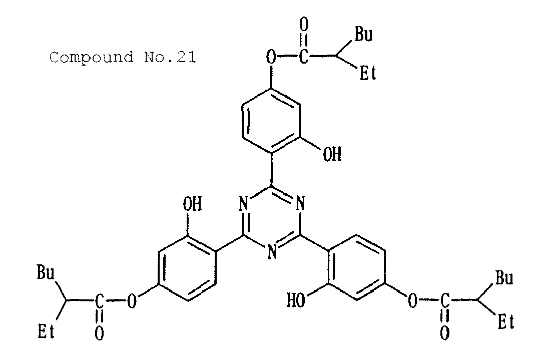

### EXAMPLE 1

### Preparation of sample (cast film)

To a 25 ml volumetric flask was added 1.25 g of polycarbonate resin (Mitsubishi Engineering-Plastics Corporation, Iupilon S-3000F) dried at 120°C for 6 hours and 6.25 mg (0.5 phr) of the compound No. 7, and dichloromethane to a marked line. The mixture was left stand at room temperature for one hour to dissolve the resin and the compound. The resulting solution was transferred to a laboratory dish (diameter: 60 mm) with a 4-ml whole pipette and was dried at room temperature for 30 minutes. The resulting film was removed from the laboratory dish, providing a PC film according to Example 1 having a thickness of 50 µm.

### Example 2 and Comparative Examples 1 and 2

A PC film according to Example 2 having a thickness of 50 µm was prepared as in Example 1, except that the compound No. 7 was replaced with the compound No. 21. Furthermore, a PC film according to Comparative Example 1 was prepared as in Example 1, except that the compound No. 7 was replaced with a comparative compound 1 described below. A PC film according to Comparative Example 2 was prepared as in Example 1, except that the compound No. 7 was not used.

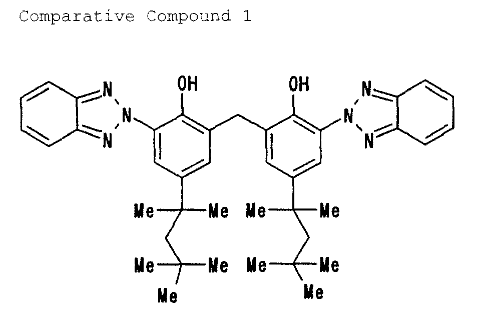

### Weatherability test method

The yellow indexes of the PC films according to the examples and the comparative examples in a sunshine weather meter (manufactured by Suga Test Instruments Co., Ltd.) (83°C) with water spray and an ATLAS xenon arc weatherometer (65°C) with water spray (in both cases, continuous irradiation and water spray for 18 minutes every 120 minutes) were measured with a multi-spectro color meter (manufactured by Suga Test Instruments Co., Ltd.) every 120 hours.

In the weatherability test using the sunshine weather meter, the yellow index was measured in transmission mode of the films. Table 1 shows the color difference (ΔE), and Table 2 shows the difference in yellow index (ΔY.I.). In the weatherability test using the xenon arc weatherometer, the yellow index was measured in reflection mode. Table 3 shows the color difference (ΔE), and Table 4 shows the difference in yellow index (ΔY.I.).

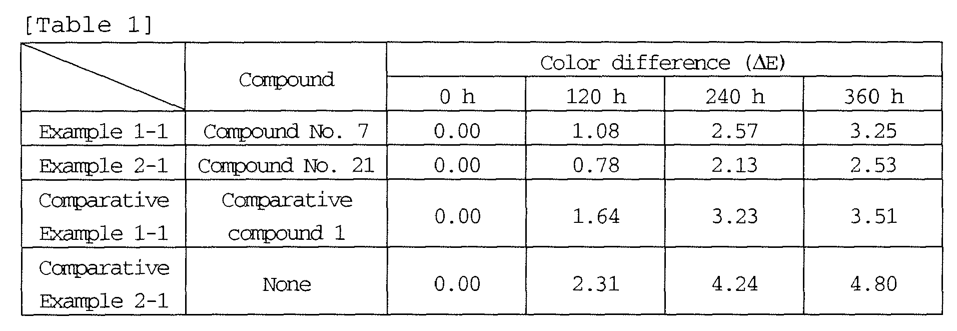

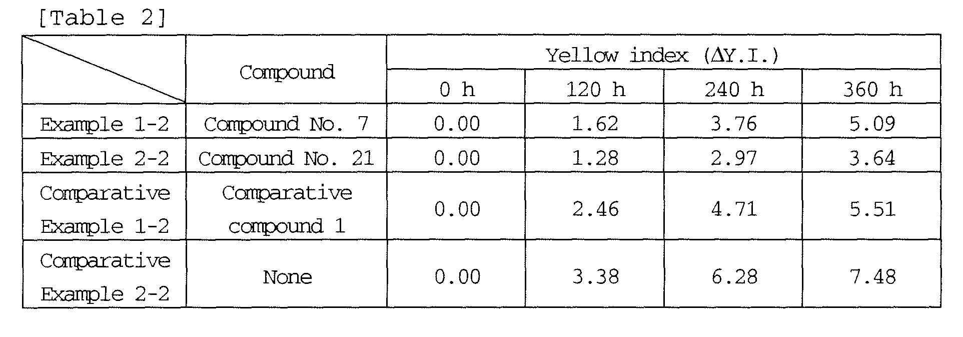

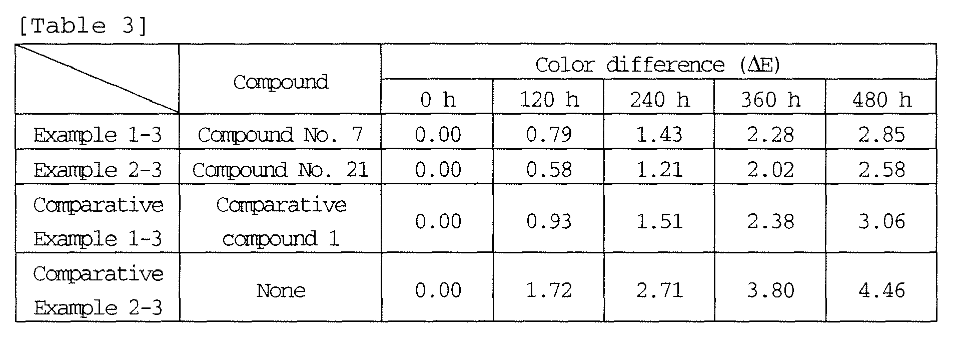

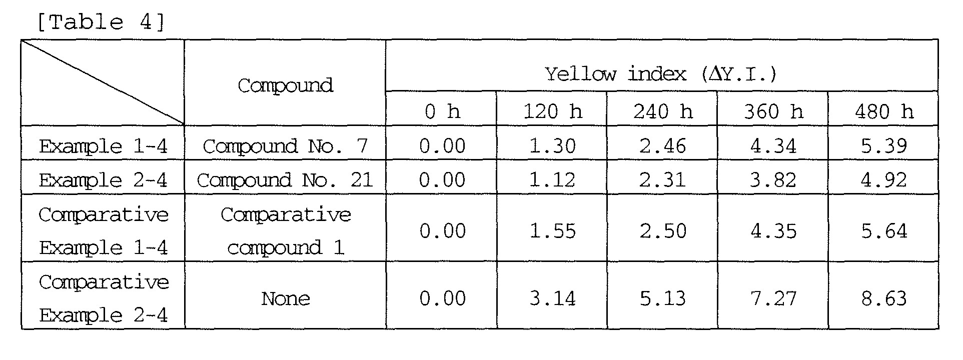

## Claims

1. A polymeric material composition comprising:
a polymeric material and a triazine compound having the general formula (I): 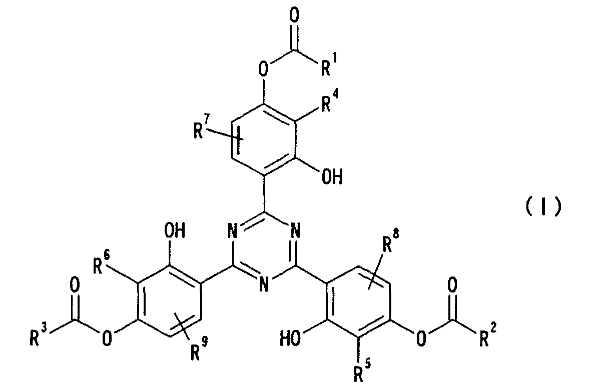 wherein R¹ to R³ independently denote a straight- or branched-chain alkyl group having 1 to 18 carbon atoms, a cycloalkyl group having 3 to 8 carbon atoms, an alkenyl group having 3 to 8 carbon atoms, or an aryl group, an alkylaryl group, or an arylalkyl group each having 6 to 18 carbon atoms, the alkyl group, the cycloalkyl group, the alkenyl group, the aryl group, the alkylaryl group, or the arylalkyl group being optionally substituted by a hydroxy group, a halogen atom, a cyano group, a nitro group, or an alkyl group or an alkoxy group each having 1 to 18 carbon atoms, and/or being optionally interrupted by an oxygen atom, a sulfur atom, a carbonyl group, an ester group, an amide group, or an imino group; R⁴ to R⁶ independently denote a hydrogen atom, a straight- or branched-chain alkyl group having 1 to 8 carbon atoms, or an alkenyl group having 3 to 8 carbon atoms; and R⁷ to R⁹ independently denote a hydrogen atom, a halogen atom, a straight- or branched-chain alkyl group having 1 to 8 carbon atoms, or an alkenyl group having 3 to 8 carbon atoms.

2. The polymeric material composition according to Claim 1, wherein R⁴, R⁵, and R⁶ in the general formula (I) are methyl groups.

3. The polymeric material composition according to Claim 2, wherein R⁷, R⁸, and R⁹ in the general formula (I) are hydrogen atoms.

4. The polymeric material composition according to Claim 1, wherein R⁴, R⁵, R⁶, R⁷, R⁸, and R⁹ in the general formula (I) are hydrogen atoms.

5. The polymeric material composition according to any one of Claims 1 to 4, wherein the polymeric material is a polycarbonate resin.
